(19) **Europäisches Patentamt / European Patent Office / Office européen des brevets**

(11) **EP 2 918 381 B1**

(12) **EUROPEAN PATENT SPECIFICATION**

(45) Date of publication and mention
of the grant of the patent:
**06.12.2017 Bulletin 2017/49**

(21) Application number: **13853097.7**

(22) Date of filing: **28.10.2013**

(51) Int Cl.:
*B26B 21/44* (2006.01)     *C08G 18/00* (2006.01)
*C08L 71/02* (2006.01)     *C08L 53/02* (2006.01)
*A61Q 19/00* (2006.01)     *A61K 8/81* (2006.01)
*A61K 8/86* (2006.01)     *A61K 8/87* (2006.01)
*C08G 18/48* (2006.01)     *C08G 18/66* (2006.01)
*C08G 18/75* (2006.01)     *C08G 18/08* (2006.01)
*C08G 65/333* (2006.01)     *A45D 27/02* (2006.01)
*A45D 27/38* (2006.01)

(86) International application number:
**PCT/JP2013/079154**

(87) International publication number:
**WO 2014/073411 (15.05.2014 Gazette 2014/20)**

(54) **LUBRICATIVE RESIN COMPOSITION**

GLEITFÄHIGE HARZZUSAMMENSETZUNG

COMPOSITION DE RÉSINE LUBRIFIANTE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **06.11.2012 JP 2012244336**

(43) Date of publication of application:
**16.09.2015 Bulletin 2015/38**

(73) Proprietors:
• **Sumitomo Seika Chemicals Co., Ltd.**
**Kako-gun, Hyogo 675-0145 (JP)**
• **Kai R & D Center Co., Ltd.**
**Seki-shi, Gifu 501-3992 (JP)**

(72) Inventors:
• **OZAWA, Hitoshi**
**Osaka-shi**
**Osaka 541-0041 (JP)**
• **MASUDA, Tsuyoshi**
**Himeji-shi**
**Hyogo 672-8076 (JP)**

• **HABA, Daisuke**
**Seki-shi**
**Gifu 501-3992 (JP)**

(74) Representative: **Webster, Jeremy Mark et al**
**Mewburn Ellis LLP**
**City Tower**
**40 Basinghall Street**
**London EC2V 5DE (GB)**

(56) References cited:
EP-A- 0 771 255          EP-A1- 1 084 012
WO-A1-2010/150875     WO-A1-2011/132615
AU-B2- 746 691          JP-A- H0 724 156
JP-A- 2005 515 868      JP-A- 2007 229 513
US-A- 5 781 997          US-A1- 2006 272 155
US-A1- 2011 146 079     US-A1- 2011 203 113
US-A1- 2012 030 947     US-A1- 2012 102 742
US-A1- 2012 102 745     US-A1- 2012 102 761
US-A1- 2012 103 928

**Description**

TECHNICAL FIELD

The present invention relates to a lubricating resin composition whose lubricity is achieved under a wet condition, especially, a lubricating resin composition for use in personal care products to be used in contact with a skin. The present invention more particularly relates to a lubricating resin composition for personal care products obtained by mixing a thermoplastic elastomer, a water-soluble polyethylene oxide and a water-absorbent modified polyalkylene oxide.

BACKGROUND ART

For personal care products such as a razor, a razor blade cartridge has conventionally been proposed, which comprises a shaving aid containing a water-soluble resin such as a polyalkylene oxide attached to a portion of the razor blade cartridge made of plastic, in order to reduce the resistive force between a portion of a razor and the facial surface, etc., (for example, Patent Document 1).

It is known that a high-molecular weight compound (water-soluble resin) obtained by reacting a polyalkylene oxide compound and a diisocyanate with each other and having a weight-average molecular weight of 10000 or higher is used as a water-soluble solidified shaving aid (Patent Document 2).

A composite may be used as a smoother (lubricating agent) for wet shaving, wherein the composite comprises a water-swellable polymer swelling and releasing various aids upon immersion of the composite in water due to mixing of a water-soluble polymer (water-soluble resin) and the water-swellable polymer (Patent Document 3).

A polymer composite is disclosed as a polymer composite used in wet shaving instruments, medical instruments, etc., wherein the polymer composite comprises a water-insoluble polymer, and a water-sensitive copolymer (water-soluble resin) obtained by polymerizing an alkylene oxide monomer and an epoxy-functional monomer (Patent Document 4).

It has been proposed that a composition obtained by combination of a thermoplastic elastomer and a water-soluble polyethylene oxide is used as a guard made of a material which can bend depending on the external form of the skin to be shaved (Patent Document 5).

WO 2010/150875A discloses a resin composition which maintains lubricity under wet conditions. The composition is produced by melt-mixing a water-soluble polyethylene oxide having a viscosity average molecular weight of 1,000,000 to 5,000,000, a modified polyalkylene oxide, and a thermoplastic resin.

PRIOR ART DOCUMENT

Patent Documents

Patent Document 1: JP 54-94961 A
Patent Document 2: JP 07-24156 A
Patent Document 3: JP 09-502632 A
Patent Document 4: JP 2004-509207 A
Patent Document 5: JP 2003-19372 A

SUMMARY OF THE INVENTION

Problem to be solved by the Invention

The razor blade cartridge described in Patent Document 1, the shaving aid described in Patent Document 2, the composite described in Patent Document 3, the polymer composite described in Patent Document 4, and the guard described in Patent Document 5 utilize elution of a water-soluble resin under a wet condition to give lubricity to each of the surfaces of the cartridge.

However, when a desired member is formed by melt-mixing a water-soluble resin and a thermoplastic resin, the water-soluble resin is scattered in blocks in the surface of the member since the compatibility between the water-soluble resin and the thermoplastic resin is low. For this reason, although lubricity at the initial stage of use is superior, the scattered water-soluble resin is dropped in blocks when it is repeatedly used, and the lubricity is lost in a short time.

The shaving aid described in Patent Document 2 and comprising the high-molecular weight compound is superior in water-solubility, and the water-soluble component thereof therefore flows out at one time by an amount equal to or more than a necessary amount. Thus, the following problems arise: that the skin becomes slimy; and that the water-soluble component of the compact drops off to cause contraction and hardening in repeated use thereof resulting in degradation of the durability thereof. Although mixing with another resin is also examined in order to improve durability, another problem arises that the compatibility therebetween is low.

A water-soluble resin such as a polyalkylene oxide generally has more excellent lubricity as the molecular weight thereof is higher, while cobwebbing thereof under a wet condition becomes more remarkable and feeling in use becomes worse, and furthermore, since the melt viscosity thereof becomes higher, it is necessary to raise the temperature for the processing. Thus, the high-molecular weight water-soluble resin is often mixed with a polymer having a low hydrophilic property and low flexibility such as polystyrene or high impact polystyrene each having a high processing temperature. The mixture thus obtained is superior in lubricity under a wet condition, while polystyrene or high impact polystyrene as such are hard, and have low flexibility and low contact property to the skin, causing therefore a problem to arise that a roughness resistant property thereof is degraded because feeling of roughness is generated on the surface thereof due to the dropping off of the water-soluble resin component in repeated use. When the water-soluble resin is increased in order to maintain the good lubricity, the swelling ratio under a wet condition is increased, and thus, when the composition is then dried in the repeated use, a problem arises that the surface of the composition is deformed to have recesses and protrusions.

Patent Document 5 proposes a use of a thermoplastic elastomer as a thermoplastic resin for improving contact property to the skin. However, compatibility between the thermoplastic elastomer and a hydrophilic polymer is low, and therefore, the water-soluble component drops off at once, causing problems to arise that touch feeling is degraded due to the resulting fine pores on the surface in repeated use thereof, and that its durability is inferior.

An object of the present invention is to provide a lubricating resin composition for personal care, wherein the composition not only maintains its superior lubricity and roughness resistant property under a wet condition even in repeated use thereof, but also has a low swelling ratio and represents low slimy feeling under a wet condition.

Means to Solve the Problems

The inventors has actively studied to solve the above problems, and as a result, found that a lubricating resin composition comprising a certain thermoplastic elastomer, a water-soluble polyethylene oxide and a water-absorbent modified polyalkylene oxide mixed therein was superior in flexibility and simultaneously has a superior durability in repeated use thereof, and the inventors has achieved the present invention.

The present invention includes the following lubricating resin compositions for personal care.

[1] A lubricating resin composition for personal care, wherein the composition comprises 100 parts by mass of a thermoplastic elastomer having durometer A hardness (HDA) of 10 to 40, 10 to 40 parts by mass of a water-soluble polyethylene oxide, and 20 to 60 parts by mass of a water-absorbent modified polyalkylene oxide.

[2] The lubricating resin composition for personal care according to [1], wherein a used amount of the water-soluble polyethylene oxide (A) and a used amount of the water-absorbent modified polyalkylene oxide (B) satisfy B $\geq$ A.

[3] The lubricating resin composition for personal care according to [1] or [2], wherein the thermoplastic elastomer is a styrene-based elastomer.

[4] The lubricating resin composition for personal care according to any one of [1] to [3], wherein the water-soluble polyethylene oxide has a viscosity-average molecular weight of 100000 to 5000000.

[5] The lubricating resin composition for personal care according to any one of [1] to [4], wherein the water-absorbent modified polyalkylene oxide is a modified polyalkylene oxide having a water absorption ability of 10 to 40 [g/g] and obtained by reaction of a polyalkylene oxide compound, a diol compound and a diisocyanate compound.

[6] The lubricating resin composition for personal care according to any one of [1] to [5], wherein the water-absorbent modified polyalkylene oxide has a ratio of a water-soluble component of 5 to 30% by mass.

Effect of the Invention

The lubricating resin composition of the present invention is superior in flexibility, and can maintain its superior lubricity under a wet condition even in repeated use thereof. In addition, the lubricating resin composition of the present invention represents a good deformation resistance under a dry condition due to its relatively low swelling ratio under a wet condition. As a result, the lubricating resin composition of the present invention is widely usable for personal care products such as wet shaving instruments typified by a razor.

BRIEF DESCRIPTION OF DRAWINGS

[Fig. 1] Fig. 1 is a schematic diagram of a method of determining a mean coefficient of friction (MIU).
[Fig. 2] Fig. 2 is a schematic diagram of a method of determining a deviation in the mean coefficient of friction (MMD).

MODES FOR CARRYING OUT THE INVENTION

The lubricating resin composition of the present invention comprises a thermoplastic elastomer, a water-soluble polyethylene oxide, and a water-absorbent modified polyalkylene oxide.

As the thermoplastic elastomer used for the lubricating resin composition of the present invention, any general styrene-based, olefin-based, PVC-based, polyester-based, urethane-based, amide-based elastomers can be used. A lightweight styrene-based elastomer having a high elasticity is suitably used as this thermoplastic elastomer. Among styrene-based elastomers, a hydrogenerated styrene-based elastomer having a low hardness is preferable, form the viewpoint of the superior weather resistance and heat resistance thereof.

As the hydrogenerated styrene-based elastomer having a low hardness, polystyrene-poly(ethylene/butylene)block-polystyrene (SEBS), polystyrene-poly(ethylene/propylene)block-polystyrene (SEPS), and the mixture thereof can be suitably used.

Examples of a commercially available product of these SEBS and SEPS include "Septon CJ103" and "Septon JS20N" (manufactured by KURARAY PLASTICS Co., Ltd.), "THERMOLAST K" (manufactured by KRAIBURG), and "Dynaron SEBS" (JSR Corporation).

The thermoplastic elastomer used in the present invention has durometer A hardness (HDA) of 10 or higher and preferably 15 or higher as a lower limit, and of 40 or lower and preferably 30 or lower as an upper limit, and has durometer A hardness (HDA) in a range of 10 to 40 and preferably 15 to 30. If a thermoplastic elastomer having HDA higher than 40 is used, the resulting lubricating resin composition has a decreased flexibility and a decreased bending property. If HDA is lower than 10, compatibility with the water-soluble polyethylene oxide and the water-absorbent modified polyalkylene oxide is low, and the resulting lubricating resin composition has decreased lubricity in repeated use thereof. When the thermoplastic elastomer is a mixture of two or more of elastomers, the mixture must have HDA in a range of 10 to 40.

The water-soluble polyethylene oxide used in the lubricating resin composition of the present invention is a water-soluble resin obtained by polymerizing ethylene oxide. This water-soluble polyethylene oxide is not particularly limited, and may be also synthesized by a known production process or may be also a commercially available product. For example, "PEO®" manufactured by SUMITOMO SEIKA CHEMICAL Co., LTD. can be used as the commercially available product.

The water-soluble polyethylene oxide preferably has a viscosity-average molecular weight of 100000 or higher and more preferably 150000 or higher as a lower limit, and preferably has a viscosity-average molecular weight of 5000000 or lower and more preferably 4000000 or lower as an upper limit, and preferably has a viscosity-average molecular weight in a range of 100000 to 5000000 and more preferably 150000 to 4000000.

If its viscosity-average molecular weight is lower than 100000, the composition may be inferior in initial lubricity. If its viscosity-average molecular weight is higher than 5000000, it may be difficult to maintain its lubricity in repeated use thereof since its compatibility with the thermoplastic resin is decreased due to its high melt viscosity. By mixing water-soluble polyethylene oxides having different viscosity-average molecular weights, the workability and feeling in use can be modified.

The water-absorbent modified polyalkylene oxide used in the present invention is, for example, a modified polyalkylene oxide obtained by reacting a polyalkylene oxide compound, a diol compound and a diisocyanate compound with each other. Such a water-absorbent modified polyalkylene oxide has a low melt viscosity and thus has good compatibility with the thermoplastic elastomer and the water-soluble polyethylene oxide.

The polyalkylene oxide compound for producing the water-absorbent modified polyalkylene oxide is a homopolymer or a copolymer comprising one, or two or more type (s) of alkylene oxide group selected from the group consisting of preferably alkylene oxide groups having 2 to 9 carbon atoms and more preferably alkylene oxide groups having 2 to 4 carbon atoms.

Preferable specific examples of the alkylene oxide group include ethylene oxide, 1,2-propylene oxide, 1,3-propylene oxide, isobutylene oxide, 1,2-butylene oxide, 2,3-butylene oxide, 1,3-butylene oxide, trimethylethylene oxide, tetramethylethylene oxide, cyclohexene oxide, oxetane, tetrahydrofuran, tetrahydropyran, 1,2-pentene oxide and 1,2-hexene oxide. The polyalkylene oxide compound may be used alone, and two or more types thereof may be also used in combination.

The polyalkylene oxide compound is preferably a polyalkylene oxide compound comprising 90% by mass or more of ethylene oxide groups and more preferably a polyalkylene oxide compound comprising 95% by mass or more of ethylene oxide groups, from the viewpoint of further improvement in lubricity of the lubricating resin composition in the initial state. Each of these ratios is a value relative to the overall mass of the polyalkylene oxide compound.

The polyalkylene oxide compound is preferably a polyalkylene oxide compound having a number-average molecular weight of 5000 to 50000, and more preferably a polyalkylene oxide compound having a number-average molecular weight of 10000 to 30000, from the viewpoint of further improvement in lubricity of the lubricating resin composition in the initial state and in its repeated use.

The "number-average molecular weight" is a value calculated by hydroxyl value method (Japanese Industrial Standards: JIS K 1557-1 (2007), Plastics - Polyurethane Material Polyol Measurement Method - Part 1: Determination Method of Hydroxyl Value) according to the following formula.

```
Number-average molecular weight = 56110 x 2 / Hydroxyl Value
```

The diol compound for producing the water-absorbent modified polyalkylene oxide is one, or two or more type (s) of diol selected from the group consisting of diols preferably having 2 to 9 carbon atoms and more preferably having 2 to 5 carbon atoms. Specific examples of the diol compound include aliphatic diol compounds such as ethylene glycol, diethylene glycol, triethylene glycol, tetraethylene glycol, propylene glycol, dipropylene glycol, trimethylene glycol, 1,3-butanediol, 2,3-butanediol, 1,4-butanediol, 1,5-pentanediol, 1,6-hexanediol and 1,9-nonanediol. Among these diol compounds, ethylene glycol and 1,4-butanediol are suitably used from the viewpoints that the obtained water-absorbent modified polyalkylene oxide is superior in water absorption ability, that elution of a water-soluble component therefrom is suppressed, and that stability thereof is superior. The diol compound may be used alone, and two or more types thereof may be also used in combination.

The used amount of the diol compound preferably is from 0.8 to 2.5 mol and more preferably from 1.0 to 2.0 mol, relative to 1 mol of the polyalkylene oxide compound, from the viewpoint of water absorption ability of the water-absorbent modified polyalkylene oxide. The molar number of the polyalkylene oxide compound can be determined by dividing the mass thereof by the number average molecular weight thereof.

The diisocyanate compound is not particularly limited only when the diisocyanate compound is a compound having two isocyanate groups (-NCO) in one same molecule. Examples thereof include aromatic diisocyanates such as 4,4'-diphenylmethane diisocyanate (MDI), 1,8-dimethylbenzol-2,4-diisocyanate and 2,4-tolylene diisocyanate (TDI); aliphatic diisocyanates such as 1, 6-hexamethylene diisocyanate (HDI); and alicyclic diisocyanates such as dicyclohexylmethane-4,4'-diisocyanate (HMDI) and 3-isocyanatomethyl-3,5,5-trimethylcyclohexyl isocyanate (IPDI). Among these diisocyanate compounds, aliphatic diisocyanates and alicyclic diisocyanates are suitably used from the viewpoints of the compatibility with the thermoplastic elastomer, suppression of elution of a water-soluble compound, and superior stability, and dicyclohexylmethane-4,4'-diisocyanate (HMDI) and 1,6-hexamethylene diisocyanate (HDI) are more suitably used. The diisocyanate compound may be used alone, and two or more types thereof may be also used in combination.

The ratio in use of each of the polyalkylene oxide compound, the diol compound, and the diisocyanate compound is determined such that the ratio of the molar number of isocyanate groups of the diisocyanate compound relative to the total molar number of terminal hydroxyl groups of the polyalkylene oxide compound and hydroxyl groups of the diol compound [R value = (-NCO group/-OH group)] is preferably in a range of 0 . 7 to 1.2 and more preferably in a range of 0.8 to 1.05, from the viewpoint of further improvement in the lubricity in repeated use, and the compatibility between the water-absorbent modified polyalkylene oxide and the thermoplastic elastomer. The molar number of the isocyanate groups of the diisocyanate compound can be determined by doubling the value obtained by dividing the mass of the diisocyanate compound by its number average molecular weight.

Examples of methods of reacting the polyalkylene oxide compound, the diol compound and the diisocyanate compound with each other include a method of dissolving or dispersing all the compounds in a reaction solvent such as toluene, xylene or dimethylformamide to cause the compounds to react; and a method of uniformly mixing all the compounds in a powder state or a solid state, and then heating the mixture to a predetermined temperature to cause the compounds to react in a molten state. From the viewpoint of the industrial implementation, preferable is a method of continuously supplying the raw materials in the molten state, and mixing the raw materials in a multi-screw extruder to cause the raw materials to react. The temperature of the reaction is preferably from 70 to 210°C.

When the water-absorbent modified polyalkylene oxide is produced, from the viewpoint of promoting the reaction, a small amount of triethylamine, triethanolamine, dibutyltin dilaurate, dioctyltin dilaurate, tin 2-ethylhexanoate, or triethylenediamine may be also added to the reaction system.

Thus, the water-absorbent modified polyalkylene oxide can be obtained by reacting the polyalkylene oxide compound, the diol compound and the diisocyanate compound. A urethane group is formed in the water-absorbent modified polyalkylene oxide from the reaction between an isocyanate group and a hydroxyl group, and an interaction acts between the urethane group and a polar group of the above-mentioned thermoplastic elastomer to improve the compatibility between the water-absorbent modified polyalkylene oxide and the thermoplastic elastomer. The water-soluble polyethylene oxide has a high compatibility due to an interaction between a polyalkylene oxide part of the water-absorbent modified polyalkylene oxide and an ethylene oxide part of the water-soluble polyethylene oxide. It can be considered that superior lubricity in the repeated use and the relatively low swelling ratio under a wet condition are achieved due to this improvement of the compatibility.

The water-absorbent modified polyalkylene oxide used in the lubricating resin composition of the present invention preferably has a water absorption ability of 10 [g/g] or higher and more preferably 15 [g/g] or higher as a lower limit, and preferably has a water absorption ability of 40 [g/g] or lower and more preferably 35 [g/g] or lower as an upper limit, and preferably has a water absorption ability in a range of 10 to 40 [g/g] and more preferably 15 to 35 [g/g]. If the water absorption ability of the water-absorbent modified polyalkylene oxide is lower than 10 [g/g], the lubricity in the initial stage of use of and in repeated use of the obtained lubricating resin composition may be degraded.

If the water absorption ability of the water-absorbent modified polyalkylene oxide is higher than 40 [g/g], the degree of maintenance of the lubricity may be lowered in repeated use of the obtained lubricating resin composition. The "water absorption ability" in the present invention is a value measured using a method described later.

The ratio of the water-soluble component of the water-absorbent modified polyalkylene oxide used in the present invention is, relative to the mass of the water-absorbent modified polyalkylene oxide as such, preferably 5% by mass or higher and more preferably 10% by mass or higher, and is preferably 30% by mass or lower and more preferably 20% by mass or lower, and is preferably in a range of 5 to 30% by mass and more preferably 10 to 20% by mass, from the viewpoint of improving lubricity of the obtained lubricating resin composition.

The "ratio of the water-soluble component" in the present invention is a value measured by a method described later. If the ratio of the water-soluble component is lower than 5% by mass, lubricity of the obtained lubricating resin composition may be degraded. If the ratio of the water-soluble component is higher than 30% by mass, the degree of maintenance of the lubricity may be lowered in repeated use of the obtained lubricating resin composition.

From the viewpoint of further improvement of each of the initial lubricity and the compatibility with the water-soluble ethylene oxide, the melt viscosity of the water-absorbent modified polyalkylene oxide used in the lubricating resin composition of the present invention is preferably from 100 to 800 [Pa·s] and more preferably from 100 to 400 [Pa·s] when the melt viscosity is measured by means of a flow tester (constant test force extrusion type capillary rheometer) (conditions: 170°C, 5.0 MPa, a die having a diameter of 1 mm $\times$ a length of 1 mm is used).

The used ratio of the water-soluble polyethylene oxide to the thermoplastic elastomer is 10 parts by mass or higher and preferably 20 parts by mass or higher, relative to 100 parts by mass of the thermoplastic elastomer. The used ratio of the water-soluble polyethylene oxide to the thermoplastic elastomer is 40 parts by mass or lower and preferably 30 parts by mass or lower, relative to 100 parts by mass of the thermoplastic elastomer.

The used ratio of the water-absorbent modified polyalkylene oxide to the thermoplastic elastomer is 20 parts by mass or higher and preferably 25 parts by mass or higher, relative to 100 parts by mass of the thermoplastic elastomer. The used ratio of the water-absorbent modified polyalkylene oxide to the thermoplastic elastomer is 60 parts by mass or lower and preferably 50 parts by mass or lower, relative to 100 parts by mass of the thermoplastic elastomer.

As to the used ratio of the water-soluble polyethylene oxide and the water-absorbent modified polyalkylene oxide to the thermoplastic elastomer, relative to 100 parts by mass of the thermoplastic elastomer, the water-soluble polyethylene oxide is in a range of 10 to 40 parts by mass and preferably 20 to 30 parts by mass, and the water-absorbent modified polyalkylene oxide is in a range of 20 to 60 parts by mass and preferably 25 to 50 parts by mass.

If the used ratio of the water-soluble polyethylene oxide is lower than 10 parts by mass relative to 100 parts by mass of the thermoplastic elastomer, initial lubricity may be inferior. If the used ratio of the water-soluble polyethylene oxide is higher than 40 parts by mass relative to 100 parts by mass of the thermoplastic elastomer, slimy feeling under a wet condition may be increased and touch feeling may be degraded, and simultaneously lubricity in repeated use may be degraded.

If the used ratio of the water-absorbent modified polyalkylene oxide is lower than 20 parts by mass relative to 100 parts by mass of the thermoplastic elastomer, lubricity in repeated use may be degraded. If the used ratio of the water-absorbent modified polyalkylene oxide is higher than 60 parts by mass relative to 100 parts by mass of the thermoplastic elastomer, swelling ratio under a wet condition may be also increased.

The used amount of the water-soluble polyethylene oxide (A) and the used amount of the water-absorbent modified polyalkylene oxide (B) preferably satisfy $B \geq A$. If the used amount of the water-soluble polyethylene oxide (A) and the used amount of the water-absorbent modified polyalkylene oxide (B) satisfy $B < A$, lubricity in repeated use may be degraded.

Examples of a method of producing the lubricating resin composition of the present invention include 1) a method wherein the thermoplastic elastomer, the water-soluble polyethylene oxide and the water-absorbent modified polyalkylene oxide are each used by a predetermined amount and mixed in advance by means of a mixing machine such as a Henschel mixer or a blender, and then the mixture is introduced to a kneader, a roll, or an extruder, and is melt-mixed; and 2) a method wherein the thermoplastic elastomer, the water-soluble polyethylene oxide and the water-absorbent modified polyalkylene oxide are each introduced by a predetermined amount to a kneader, a roll, or an extruder by means of a metering feeder, and are melt-mixed.

A twin-screw extruder is suitably used as the machine for performing the melt-mixing process, the melting process and/or the reaction process, from the viewpoint of its superior mixability for each of the components.

After the melt-mixing process or the reaction process, the mixture may be also molded into a desired shape such as a pellet, a sheet, a bar or a fiber by executing injection molding or extrusion molding. The lubricating resin composition of the present invention may thus have the figure of a raw material such as a pellet or may have the figure of a desired compact such as a sheet, a bar or a fiber.

When the lubricating resin composition according to the present invention is produced, a stabilizer may be also added from the viewpoint of preventing resolution of each of the components and the obtained lubricating resin composition; a UV absorber may be also added from the viewpoint of improving the weather resistance of the obtained lubricating resin composition; a pigment, or a coloring material may be also added from the viewpoint of coloring the obtained lubricating resin composition; and a soap basis material may be also added from the viewpoint of imparting foamability to the obtained lubricating resin composition.

The lubricating resin composition of the present invention is useful for applications in which achievement of lubricity under a wet condition is necessary, for example, for personal care applications such as wet shaving instruments typified by a razor.

For example, when the lubricating resin composition of the present invention is used in a wet shaving instrument (razor), the lubricating resin composition constitutes a so-called shaving aid unit which is arranged in substantially parallel to a razor blade in a surface for attaching the razor blade in the razor or its cartridge. Thereby, the razor, or cartridge thereof, comprising the lubricating resin composition of the present invention can maintain its superior lubricity under a wet condition even in its repeated use. The shaving aid unit in the razor or cartridge thereof has relatively low swelling ratio under a wet condition, and thus represents a good deformation resistance under a dry condition. In addition, the shaving aid unit has a flexibility and a bending property, and therefore has a good feeling and touch feeling.

EXAMPLES

The present invention will be described below in further detail by Examples and Comparative Examples. However, the present invention is not limited to these.

[Evaluation Methods]

Various measurements and evaluations were carried out according to the following methods for the water absorption ability, the ratio of the water-soluble component, and the melt viscosity of the water-absorbent modified polyalkylene oxide described in each of Production Examples, and also the viscosity-average molecular weight of the water-soluble polyethylene oxide, and the durometer A hardness (HDA) of the lubricating resin composition, and the swelling ratio, the reduction ratio (thickness variation), the slimy feeling and the friction physical property of a sheet of the lubricating resin composition in each of Examples and Comparative Examples.

(1) Water Absorption Ability (Water-absorbent modified polyalkylene oxide)

The water absorption ability of the water-absorbent modified polyalkylene oxide was measured according to the following method.

About 1 [g] of the water-absorbent modified polyalkylene oxide was weighed (A [g]), and then was immersed in 100 [mL] of ion exchange water measured in a 200-mL beaker under room temperature (22°C) for 24 hours for gelation. The gel was then filtered with a 200-mesh wire gauze (pore size: 75 $\mu$m) and the mass thereof (B [g]) was measured. The water absorption ability was calculated according to the following formula.

$$\texttt{Water absorption ability [g/g] = B/A}$$

(2) Ratio of Water-Soluble Component

The gel obtained in the measurement of " (1) Water Absorption Ability" was dried in a hot air dryer set at 105°C to a constant weight (C [g]), and the ratio of the water-soluble component was calculated according to the following formula. "A" is defined in the same manner as "A" in the measurement of " (1) Water Absorption Ability".

$$\texttt{Ratio of water-soluble component [\% by mass] = (A-C)/A × 100}$$

(3) Melt Viscosity

The measurement was executed for 1.5 g of the water-absorbent modified polyalkylene oxide by means of a flow tester (constant test force extrusion type capillary rheometer) (manufactured by Shimadzu Corporation, Model: CFT-500C) under the following conditions.

Load: 5.0 MPa
Measurement Temperature: 170°C
Die Diameter: 1 mm
Die Length: 1 mm

(4) Viscosity-average molecular weight of water-soluble polyethylene oxide

The viscosity-average molecular weight of the water-soluble polyethylene oxide is determined according to the following viscosity formula.

[Formula 1]

$$[\eta] = K \times M^a$$

In the above formula, $[\eta]$ represents a limiting viscosity, K and a each represent coefficients determined by types of a solvent and a polymer, and M represents a viscosity-average molecular weight.

The limiting viscosity $[\eta]$ was calculated by measuring each specific viscosity $\eta^{sp}$ of solutions having various polymer concentrations c (g/dl) in pure water at 35°C by means of an Ostwald viscometer, and extrapolating the polymer concentration c into 0 on the basis of a relation between a reduced viscosities and the polymer concentrations c, wherein the reduced viscosities were obtained by dividing the specific viscosity by the polymer concentration to obtain a reduced viscosity ($\eta^{sp}/c$). Subsequently, the viscosity-average molecular weight of the water-soluble polyethylene oxide was determined by applying $6.4 \times 10^{-5}$ (g/dl) and 0.82 as values of K and a in pure water, respectively.

(5) Durometer A hardness (HDA)

According to "Testing Methods for Durometer Hardness of Plastics" described in Japanese Industrial Standard: JIS K 7215 (1986) (which corresponds to International Standard: ISO/DIS 868 Plastics and ebonite-Determination of indentation hardness by means of a durometer (Shore hardness)), a durometer A hardness refers to "Hardness" measured in "Durometer type A", and was measured while the thickness of the resin composition was 6 mm or more in lamination on a durometer holding stand. When HDA of the lubricating resin composition is less than 75, it can be determined that there is sufficiently flexibility since a shaving aid can bend in accordance with a shape of skin to be shaved by a razor.

(6) Bending test

A sheet of the lubricating resin composition obtained in each of Examples and Comparative Examples was cut into 2 cm (width: W) x 5 cm (length: L) to obtain a measurement sample.

A part of 1 cm (remaining part: 4 cm) from its edge part (short side having a width of 2 cm) in the measurement sample was bent at 90 degrees, and the bent part was evaluated according to the following standard.

In addition, the similar evaluation was separately carried out, in which the same sample was used and the bend angle was changed to 180 degrees.

(Evaluation standard)

A: A bent part is not whitened and returns to its original shape (a restitution ability exists).
B: A bent part is whitened, but returns to its original shape (a restitution ability exists).
C: A bent part is whitened, and does not return to its original shape (a restitution ability does not exist).

(7) Swelling ratio and reduction ratio

The sheets of the lubricating resin composition obtained in Examples and Comparative Examples were each cut into a sheet of 2 cm (W) × 5 cm (L) to be used as a measurement sample.

The mass (E [g]) of the measurement sample was measured, and the sample was then immersed in 100 [mL] of ion exchange water measured in a 200-mL beaker under room temperature (22°C) for predetermined times (0.5 hours , 8 hours, and 12 hours) to swell. The measurement sample was then taken out, and the water on the sample surface was wiped off with a paper towel to measure the mass (F [g]) thereof. When the swelling ratio after 12 hours is 160% or lower, it can be determined that the variation in dimensions due to swelling is small.

$$\text{Swelling Ratio [\%]} = (F - E)/E \times 100$$

Each of the samples after the swelling ratio measurement for the predetermined times was left under room temperature (22°C) for 24 hours, and then its mass (G [g]) was measured.

$$\text{Reduction ratio [\%]} = (E - G)/E \times 100$$

When the reduction ratio is ± less than 2% ("minus" means a case when the mass is increased), it can be determined that its deformation ratio in use is small.

(8) Friction Physical Property

The sheets of the lubricating resin composition obtained in Examples and Comparative Examples were each cut into a sheet of 2 cm (W) × 5 cm (L) to be used as a measurement sample.

0.2 mL of ion exchange water was added dropwise onto each of the measurement samples, and the measurement samples were left still for 30 seconds. Then, the coefficient of friction $\mu$ was monitored by means of a friction tester (manufactured by KATO TECH Co., LTD., Model: KES-SE) under the following test conditions.

Sensor: silicone
Load: 50 [g]
Velocity: 10 [mm/sec]

(i) Mean Coefficient of Friction (MIU) (Lubricity)

The mean coefficient of friction has correlation with ease of slipping and resistance against slipping felt in rubbing the surface. The surface is more difficult to slip on, as the value become greater.

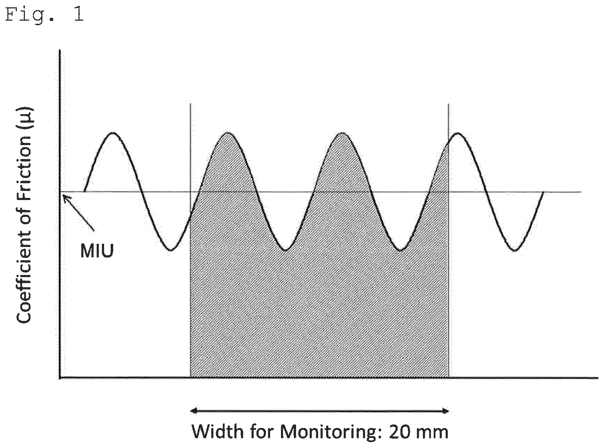

A schematic view is shown in Fig. 1 for obtaining a mean coefficient of friction (MIU) from a result of the monitoring of coefficient of friction $\mu$.

As shown in Fig. 1, the surface of the measurement sample is scanned for monitoring coefficient of friction $\mu$ of the surface. The coefficient of friction $\mu$ is integrated for a width of 20 mm for the monitoring (shaded area of Fig. 1). The mean coefficient of friction (MIU) is obtained by dividing the integral value by the width (20 mm) for the monitoring.

It can be determined that its lubricity is superior when the value of MIU is 0.30 or lower, and its lubricity is best when the value is 0.25 or lower.

(ii) Deviation in Mean Coefficient of Friction (MMD) (Roughness Resistant Property)

The deviation in the mean coefficient of friction has correlation with smoothness and roughness felt in rubbing the surface. The surface is rougher, as this value becomes greater.

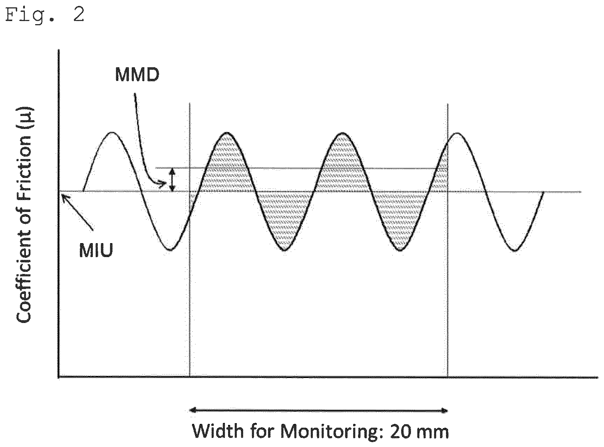

A schematic diagram is shown in Fig. 2 for determining the deviation in the mean coefficient of friction (MMD) from results of the monitoring of the coefficient of friction.

As shown in Fig. 2, in the width of 20 mm for monitoring, an absolute value of a difference between the mean coefficient of friction (MIU) and the coefficient of friction $\mu$ is integrated (shaded area of Fig. 2). The deviation in the mean coefficient of friction (MMD) is obtained by dividing the integral value by the width for the monitoring (20 mm).

It can be determined that the smoothness of the surface is good when the value of MMD is from 0.009 to 0.015.

When the value of MMD is 0.015 or lower, the value is in such a range that no problem arises in the practical use. When the value is 0.010 or lower, the smoothness of the surface is good. When the value of MMD is 0.005 or lower, the smoothness of the surface is best.

(iii) Durability test

Also with each of the samples after measurement of their swelling ratios and reduction ratios for the predetermined times, its mean coefficient of friction and its deviation were determined.

Production Example 1: Production of Water-absorbent Modified Polyalkylene Oxide

One hundred parts by mass of a fully dehydrated polyethylene oxide having a number average molecular weight of 20000, 0.90 parts by mass of 1,4-butanediol and 0.1 part by mass of dioctyltin dilaurate were put at these ratios into storage tank A equipped with a stirrer and held at 80°C, and were stirred under a nitrogen gas atmosphere to obtain a uniform mixture. Separately, dicyclohexylmethane-4,4'-diisocyanate was put into storage tank B held at 30°C, and was stored in a nitrogen gas atmosphere.
The mixture in storage tank A was continuously introduced at a rate of 500 [g/min], and dicyclohexylmethane-4,4'-disisocyanate in storage tank B was continuously introduced at a rate of 19.4 [g/min], to a twin-screw extruder set at 110 to 140°C (R value = 1.00) by means of a metering pump, and they were mixed in the extruder to perform a reaction. A strand was then discharged through an exit of the extruder, and was pelletized (into blocks of 4 × 4 × 2.5 mm) by means of a pelletizer to obtain a water-absorbent modified polyalkylene oxide.
The obtained water-absorbent modified polyalkylene oxide had a water absorption ability of 25 [g/g], a ratio of the water-soluble component of 15.5 [% by mass], and a melt viscosity of 320 [Pa·s].

Production Example 2: Production of Water-absorbent Modified Polyalkylene Oxide

An ethylene oxide/propylene oxide (mass ratio: 90/10) copolymer having a number average molecular weight of 15000 was introduced at a rate of 250 [g/min], and ethylene glycol heated to 40°C was introduced at a rate of 2.1 [g/min], to a single-screw extruder having a diameter of 40 mm (L/D = 40, preset temperature: 90°C), and these were melt-mixed.
The mixture obtained from a discharge opening (the mixture was discharged in a uniform and molten state, and it was confirmed in LC analysis that the mixture was mixed at the charge ratio) was continuously introduced to a hopper port (preset temperature: 80°C) of a twin-screw extruder having a diameter of 30 mm (L/D = 41.5). Simultaneously, dioctyltin dilaurate was introduced to the hopper port of the twin-screw extruder at a rate of 0.5 [g/min].
Separately, dicyclohexylmethane-4,4'-diisocyanate adjusted to 30°C was introduced to a screw barrel section situated on the downstream side of the hopper port of the twin-screw extruder at a rate of 12.4 [g/min] (R value = 0.95), and was thereby caused to continuously react under a nitrogen gas atmosphere (preset temperature: 180°C). A strand obtained from the exit of the twin-screw extruder was cooled, and was then pelletized (into blocks of 4 × 4 × 2.5 mm) by means of a pelletizer to obtain a water-absorbent modified polyalkylene oxide.
The obtained water-absorbent modified polyalkylene oxide had a water absorption ability of 20 [g/g], a ratio of the water-soluble component of 11.3 [% by mass], and a melt viscosity of 150 [pa·s].

Example 1

A thermoplastic elastomer (SEBS/SEPS, manufactured by KRAIBURG, THERMOLAST K TF23ADG, Durometer A hardness (HDA) = 30), a mixture of "PEO® 18P" (manufactured by SUMITOMO SEIKA CHEMICAL Co., LTD., viscosity-average molecular weight: 4500000) and "PEO® 8P" (manufactured by SUMITOMO SEIKA CHEMICAL Co., LTD., viscosity-average molecular weight: 2000000) at the mixed ratio of 1/1 as a water-soluble polyethylene oxide, and the water-absorbent modified polyalkylene oxide obtained by the same process as in Production Example 1 were introduced into a twin-screw extruder, which had a diameter of 28 mm (L/D = 40) and was set at 180°C, at rate of 6 [kg/hr], 1.2 [kg/hr] and 1.5 [kg/hr], respectively. As a result, a lubricating resin composition was obtained. The compositional ratios of the lubricating resin composition are shown in Table 1.
The obtained lubricating resin composition was molded into a 10 cm × 10 cm × 0.1 cm sheet by means of a hot press (manufactured by Gonno Hydraulic Press Manufacturing Co., Ltd., 40t press, pressure: 4.9 MPa·G) set at 180°C.
The obtained sheet of the lubricating resin composition was cut into a predetermined size, and the above various measurements and evaluations were carried out therefor. The results thereof are shown in Table 3.

Example 2

A thermoplastic elastomer (SEPS, manufactured by KURARAY PLASTICS Co., Ltd., Septon CJ103, Durometer A hardness (HDA) = 15), "PEO® 8P" (manufactured by SUMITOMO SEIKA CHEMICAL Co., LTD., viscosity-average molecular weight: 2000000) as a water-soluble polyethylene oxide, and the water-absorbent modified polyalkylene oxide obtained by the same process as in Production Example 2 were introduced into a twin-screw extruder, which had a diameter of 28 mm (L/D = 40) and was set at 180°C, at rate of 8 [kg/hr], 2 [kg/hr] and 2.4 [kg/hr], respectively. As a result, a lubricating resin composition was obtained. The compositional ratios of the lubricating resin composition are shown in Table 1.
The obtained lubricating resin composition was molded into a 10 cm × 10 cm × 0.1 cm sheet by means of a hot press (manufactured by Gonno Hydraulic Press Manufacturing Co., Ltd., 40t press, pressure: 4.9 MPa·G) set at 180°C.
The obtained sheet of the lubricating resin composition was cut into a predetermined size, and the above various types of measurement and evaluation were executed therefor. The results thereof are shown in Table 3.

Example 3

A thermoplastic elastomer (SEBS/SEPS, manufactured by KRAIBURG, THERMOLAST K TF23ADG, Durometer A hardness (HDA) = 30), a mixture of "PEG®18P" (manufactured by SUMITOMO SEIKA CHEMICAL Co., LTD., viscosity-average molecular weight: 4500000) and "PEO® 8P" (manufactured by SUMITOMO SEIKA CHEMICAL Co., LTD., viscosity-average molecular weight: 2000000) at the mixed ratio of 3/1 as a water-soluble polyethylene oxide, and the water-absorbent modified polyalkylene oxide obtained by the same process as in Production Example 1 were introduced into a twin-screw extruder, which had a diameter of 28 mm (L/D = 40) and was set at 180°C, at rate of 9 [kg/hr], 1.8 [kg/hr] and 2.7 [kg/hr], respectively. As a result, a lubricating resin composition was obtained. The compositional ratios of the lubricating resin composition are shown in Table 1.
The obtained lubricating resin composition was molded into a 10 cm × 10 cm × 0.1 cm sheet by means of a hot press (manufactured by Gonno Hydraulic Press Manufacturing Co., Ltd., 40t press, pressure: 4.9 MPa·G) set at 180°C.
The obtained sheet of the lubricating resin composition was cut into a predetermined size, and the above various types of measurement and evaluation were executed therefor. The results thereof are shown in Table 3.

Example 4

A thermoplastic elastomer (SEBS/SEPS, manufactured by KRAIBURG, THERMOLAST K TF23ADG, Durometer A hardness (HDA) = 30), a mixture of "PEO® 18P" (manufactured by SUMITOMO SEIKA CHEMICAL Co., LTD., viscosity-average molecular weight: 4500000), "PEO®8P" (manufactured by SUMITOMO SEIKA CHEMICAL Co., LTD., viscosity-average molecular weight: 2000000) and "PEO® 1P" (manufactured by SUMITOMO SEIKA CHEMICAL Co., LTD., viscosity-average molecular weight: 200000) at the mixed ratio of 1/1/1 as a water-soluble polyethylene oxide, and the water-absorbent modified polyalkylene oxide obtained by the same process as in Production Example 2 were introduced into a twin-screw extruder, which had a diameter of 28 mm (L/D = 40) and was set at 180°C, at rate of 5 [kg/hr], 1.5 [kg/hr] and 2 [kg/hr], respectively. As a result, a lubricating resin composition was obtained. The compositional ratios of the lubricating resin composition are shown in Table 1.
The obtained lubricating resin composition was molded into a 10 cm × 10 cm × 0.1 cm sheet by means of a hot press (manufactured by Gonno Hydraulic Press Manufacturing Co., Ltd., 40t press, pressure: 4.9 MPa·G) set at 180°C.
The obtained sheet of the lubricating resin composition was cut into a predetermined size, and the above various types of measurement and evaluation were executed therefor. The results thereof are shown in Table 3.

Comparative Example 1

A lubricating resin composition was obtained in the same process as in Example 1 except that the added amount of the water-absorbent modified polyalkylene oxide obtained by the same process as in Production Example 1 was changed to 0.6 [kg/hr]. The compositional ratios thereof are shown in Table 2, and the results thereof are shown in Table 4.

Comparative Example 2

A lubricating resin composition was obtained in the same process as in Example 2 except that the added amount of the water-soluble polyethylene oxide was changed to 0.4 [kg/hr], and that the added amount of the water-absorbent modified polyalkylene oxide in Production Example 2 was changed to 4 [kg/hr]. The compositional ratios thereof are shown in Table 2, and the results thereof are shown in Table 4.

Comparative Example 3

A lubricating resin composition was obtained in the same process as in Example 3 except that the added amount of the water-soluble polyethylene oxide was changed to 4.5 [kg/hr]. The compositional ratios thereof are shown in Table 2, and the results thereof are shown in Table 4.

Comparative Example 4

A lubricating resin composition was obtained in the same process as in Example 4 except that the thermoplastic elastomer was changed to a high impact polystyrene [abbreviation: HIPS, 476L manufactured by BASF, HDA>99], and that the set temperatures of the twin-screw extruder having a diameter of 28 mm and the hot press were each changed to 220°C. The compositional ratios thereof are shown in Table 2, and the results thereof are shown in Table 4.

Comparative Example 5

A lubricating resin composition was obtained in the same process as in Example 4 except that the water-absorbent modified polyalkylene oxide was not added. The compositional ratios thereof are shown in Table 2, and the results thereof are shown in Table 4.

Comparative Example 6

A lubricating resin composition was obtained in the same process as in Example 4 except that the water-soluble polyethylene oxide was not added. The compositional ratios thereof are shown in Table 2, and the results thereof are shown in Table 4.

[Table 1]

| Example | Thermoplastic Elastomer | | | Water-soluble Polyethylene Oxide | | | Water-absorbent Modified Polyalkylene Oxide | | | | |
|---|---|---|---|---|---|---|---|---|---|---|---|
| | Type [1) | Durometer A Hardness | Partsby mass | Type (PEO) | Viscosity-average Molecular Weight | Parts by mass | Type | Water Absorption Ability [g/g] | Ratio of Water-soluble Component [%] | Melt Viscosity [Pa·s] | Partsby mass |
| 1 | SEBS/ SEPS | 30 | 100 | 18P/ 8P | 4500000/2000000 | 10/10 | Production Example 1 | 25 | 15.5 | 320 | 25 |
| 2 | SEPS | 15 | 100 | 8P | 2000000 | 25 | Production Example 2 | 20 | 11.3 | 150 | 30 |
| 3 | SEBS/ SEPS | 30 | 100 | 18P/ 8P | 4500000/2000000 | 15/5 | Production Example 1 | 25 | 15.5 | 320 | 30 |
| 4 | SEBS/ SEPS | 30 | 100 | 18P/ 8P/1P | 4500000/2000000 /200000 | 10/10/10 | Production Example 2 | 20 | 11.3 | 150 | 40 |
| 1) SEBS: Polystyrene-poly(ethylene/butylene)block-polystyrene, SEPS: Polystyrene-poly(ethylene/propylene)block-polystyrene | | | | | | | | | | | |

[Table 2]

| Comparative Example | Thermoplastic Elastomer | | | Water-soluble Polyethylene Oxide | | | Water-absorbent Modified Polyalkylene Oxide | | | | |
|---|---|---|---|---|---|---|---|---|---|---|---|
| | Type [1] | Durometer A Hardness | Parts by mass | Type (PEO) | Viscosity-average Molecular Weight | Parts by mass | Type | Water Absorption Ability [g/g] | Ratio of Water-soluble Component [%] | Melt Viscosity [Pa·s] | Parts by mass |
| 1 | SEBS/ SEPS | 30 | 100 | 18P/ 8P | 4500000/2000000 | 10/10 | Production Example 1 | 25 | 15.5 | 320 | 10 |
| 2 | SEPS | 15 | 100 | 8P | 2000000 | 5 | Production Example 2 | 20 | 11.3 | 150 | 50 |
| 3 | SEBS/ SEPS | 30 | 100 | 18P/ 8P | 4500000/2000000 | 37.5/12.5 | Production Example 1 | 25 | 15.5 | 320 | 30 |
| 4 | HIPS | >99 | 100 | 18P/ 8P/1P | 4500000/2000000 /200000 | 10/10/10 | Production Example 2 | 20 | 11.3 | 150 | 40 |
| 5 | SEBS/ SEPS | 30 | 100 | 18P/ 8P/1P | 4500000/2000000 /200000 | 10/10/10 | - | - | - | - | - |
| 6 | SEBS/ SEPS | 30 | 100 | - | - | - | Production Example 1 | 20 | 15.5 | 150 | 40 |

1) SEBS: Polystyrene-poly(ethylene/butylene)block-polystyrene, SEPS: Polystyrene-poly(ethylene/propylene)block-polystyrene HIPS: High impact polystyrene

[Table 3]

| Example | Bending Test | | Swelling Ratio [%] | | | Reduction Ratio [%] | | | Mean Coefficient of Friction (MIU) | | | | Deviation in Mean Coefficient of Friction (MMD) | | | |
|---|---|---|---|---|---|---|---|---|---|---|---|---|---|---|---|---|
| | 90 Degrees | 180 Degrees | Water Immersion Time 0.5 [hr] | Water Immersion Time 8[hr] | Water Immersion Time 12 [hr] | Water Immersion Time 0,5 [hr] | Water Immersion Time 8[hr] | Water Immersion Time 12 [hr] | Water Immersion Time 0[hr] | Water Immersion Time 0.5 [hr]→ Drying | Water Immersion Time 8[hr] → Drying | Water Immersion Time 12 [hr]→ Drying | Water Immersion Time 0[hr] | Water Immersion Time 0.5 [hr]→ Drying | Water Imersion Time 8 [hr]→ Drying | Water Immersion Time 12 [hr]→ Drying |
| 1 | A | A | 85 | 117 | 138 | -0.1 | -0.2 | -0.3 | 0.18 | 0.22 | 0.23 | 0.25 | 0.007 | 0.011 | 0.012 | 0.014 |
| 2 | A | A | 105 | 156 | 160 | -0.3 | -0.7 | -0.8 | 0.12 | 0.14 | 0.18 | 0.22 | 0.005 | 0.007 | 0.011 | 0.013 |
| 3 | A | A | 95 | 145 | 150 | -0.3 | -0.6 | -0.7 | 0.13 | 0.16 | 0.25 | 0.25 | 0.006 | 0.008 | 0.009 | 0.009 |
| 4 | A | A | 125 | 155 | 158 | -0.4 | -0.8 | -0.8 | 0.14 | 0.16 | 0.25 | 0.25 | 0.006 | 0.008 | 0.011 | 0.012 |

[Table 4]

| Comparative Example | Bending Test | | Swelling Ratio [%] | | | Reduction Ratio [%] | | | Mean Coefficient of Friction (MIU) | | | | Deviation in Mean Coefficient of Friction (MMD) | | | |
|---|---|---|---|---|---|---|---|---|---|---|---|---|---|---|---|---|
| | 90 Degrees | 180 Degrees | Water Immersion Time 0.5 [hr] | Water Immersion Time 8 [hr] | Water Immersion Time 12 [hr] | Water Immersion Time 0.5 [hr] | Water Immersion Time 8 [hr] | Water Immersion Time 12 [hr] | Water Immersion Time 0 [hr] | Water Immersion Time 0.5 [hr]→ Drying | Water Immersion Time 8 [hr]→ Drying | Water Immersion Time 12 [hr]→ Drying | Water Immersion Time 0 [hr] | Water Immersion Time 0.5 [hr]→ Drying | Water Immersion Time 8 [hr]→ Drying | Water Immersion Time 12 [hr]→ Drying |
| 1 | A | A | 55 | 85 | 90 | -0.3 | 0.7 | 0.8 | 0.18 | 0.22 | 0.32 | 0.44 | 0.007 | 0.012 | 0.025 | 0.035 |
| 2 | A | B | 125 | 188 | 195 | -2.3 | -3.2 | -4.5 | 0.14 | 0.16 | 0.23 | 0.28 | 0.008 | 0.012 | 0.019 | 0.028 |
| 3 | A | B | 135 | 190 | 205 | -1.8 | -2.3 | -2.5 | 0.18 | 0.25 | 0.38 | 0.48 | 0.006 | 0.014 | 0.022 | 0.032 |
| 4 | C | C | 103 | 122 | 128 | -0.3 | -0.6 | -0.6 | 0.14 | 0.16 | 0.26 | 0.38 | 0.007 | 0.018 | 0.024 | 0.028 |
| 5 | A | A | 131 | 137 | 128 | 2.1 | 13.7 | 18.2 | 0.21 | 0.33 | 0.66 | 0.68 | 0.023 | 0.04 | 0.12 | 0.14 |
| 6 | A | A | 123 | 152 | 155 | -0.5 | -1.2 | -1.5 | 0.25 | 0.26 | 0.35 | 0.41 | 0.008 | 0.05 | 0.06 | 0.08 |

As is apparent from the results shown in Tables 3 and 4, the lubricating resin compositions of the present invention have small variation in dimension due to swelling, and maintain the lubricity thereof and have no variation in roughness resistant property even under a wet condition, under a dry condition or in repeated use thereof, wherein the compositions comprise each certain amounts of a thermoplastic elastomer having durometer A hardness (HDA) of 10 to 40, a water-soluble polyethylene oxide and a water-absorbent modified polyalkylene oxide.

In Comparative Example 1, the amount of the water-absorbent modified polyalkylene oxide is not within the range of 20 to 60 parts by mass, and 10 parts by mass of the water-absorbent modified polyalkylene oxide is contained. In this case, after water immersion for 12 hours, the mean coefficient of friction and deviation in the mean coefficient of friction of the dry sample increased, and the lubricity and roughness resistant property thereof were low.

In Comparative Example 2, the amount of the water-soluble polyethylene oxide is not within the range of 10 to 40 parts by mass, and 5 parts by mass of the water-soluble polyethylene oxide (PEO® 18P) is contained. In this case, the swelling ratio and reduction ratio after water immersion for 12 hours increased. The result of 180 degree-bending test was B, and thus the bending property thereof was lowered.

In Comparative Example 3, the amount of the water-soluble polyethylene oxide is not within the range of 10 to 40 parts by mass, and 37.5/12.5 parts by mass of the water-soluble polyethylene oxide (PEO® 18P/PEO® 8P) is contained. In this case, the swelling ratio and reduction ratio after water immersion for 12 hours increased. The result of 180 degree-bending test was B, and thus the bending property thereof was degraded. In addition, after water immersion for 12 hours, the mean coefficient of friction and deviation in the mean coefficient of friction of the dry sample increased, and the lubricity and roughness resistant property thereof were low.

In Comparative Example 4, the HDA of the thermoplastic elastomer is not within the range of 10 to 40, and the thermoplastic elastomer (HIPS) having a HDA > 99 is contained. In this case, the mean coefficient of friction and deviation in the mean coefficient of friction after water immersion for 12 hours increased, and the lubricity and roughness resistant property thereof were lowered. The results of 90 degree- and 180 degree-bending tests were each C, and thus the bending property thereof was degraded.

In Comparative Example 5, the amount of the water-absorbent modified polyalkylene oxide is not within the range of 20 to 60 parts by mass, and the water-absorbent modified polyalkylene oxide is not contained. In this case, the reduction ratio after water immersion for 12 hours was low. In addition, after water immersion for 12 hours, the mean coefficient of friction and deviation in the mean coefficient of friction of the dry sample increased, and the lubricity and roughness resistant property thereof were lowered.

In Comparative Example 6, the amount of the water-soluble polyethylene oxide is not within the range of 10 to 40 parts by mass, and the water-soluble polyethylene oxide is not contained. In this case, after water immersion for 12 hours, the mean coefficient of friction and deviation in the mean coefficient of friction of the dry sample increased, and the lubricity and roughness resistant property thereof were lowered.

INDUSTRIAL APPLICABILITY

The lubricating resin composition according to the present invention maintains its lubricity even under a wet or dry condition or in repeated use thereof, and can thus be suitably used in a wide field in which lubricity under a wet condition is necessary, such as wet shaving instruments typified by a razor.

**Claims**

1. A lubricating resin composition for personal care, wherein the composition comprises 100 parts by mass of a thermoplastic elastomer having durometer A hardness (HDA) of 10 to 40 measured according to Japanese Industrial Standard JIS K 7215 (1986), 10 to 40 parts by mass of a water-soluble polyethylene oxide, and 20 to 60 parts by mass of a water-absorbent modified polyalkylene oxide.

2. The lubricating resin composition for personal care according to claim 1, wherein a used amount of the water-soluble polyethylene oxide (A) and a used amount of the water-absorbent modified polyalkylene oxide (B) satisfy $B \geq A$.

3. The lubricating resin composition for personal care according to claim 1 or 2, wherein the thermoplastic elastomer is a styrene-based elastomer.

4. The lubricating resin composition for personal care according to any one of claims 1 to 3, wherein the water-soluble polyethylene oxide has a viscosity-average molecular weight, M, of 100000 to 5000000, measured according to the formula:

$$[\eta] = K \times M^a$$

in which $[\eta]$ represents limiting viscosity, K is a coefficient whose value is $6.4 \times 10^{-5}$ (g/dl) and a is a coefficient whose value is 0.82, the limiting viscosity $[\eta]$ being calculable by measuring specific viscosities $\eta_{sp}$ of solutions having various polymer concentrations c (g/dl) in pure water at 35°C by means of an Ostwald viscometer, dividing the specific viscosity by the polymer concentration to obtain a reduced viscosity ($\eta^{sp}/C$), the limiting viscosity then being determinable on the basis of the relationship between reduced viscosity and polymer concentration c, when polymer concentration is extrapolated to 0.

5. The lubricating resin composition for personal care according to any one of claims 1 to 4, wherein the water-absorbent modified polyalkylene oxide is a modified polyalkylene oxide having a water absorption ability of 10 to 40 [g/g] and obtained by reaction of a polyalkylene oxide compound, a diol compound and a diisocyanate compound, wherein the water absorption ability is the ratio B/A (g/g), determinable by taking about 1g of the water-absorbent modified polyalkylene oxide and weighing it to find its mass, A (g), immersing the weighed modified polyalkylene oxide in 100 mL of ion exchange water measured in a 200 mL beaker at 22°C for 24 hours to form a gel, filtering the gel with a 200-mesh wire gauze, having a pore size of 75 $\mu$m and measuring the mass, B (g) of the gel.

6. The lubricating resin composition for personal care according to any one of claims 1 to 5, wherein the water-absorbent modified polyalkylene oxide has a ratio of a water-soluble component of 5 to 30% by mass, wherein the ratio of water-soluble component [% by mass] is given by the formula

$$100 \ (A-C)/A$$

and A and C are determinable by taking about 1g of the water-absorbent modified polyalkylene oxide and weighing it to find its mass, A (g), immersing the weighed modified polyalkylene oxide in 100 mL of ion exchange water measured in a 200 mL beaker at 22°C for 24 hours to form a gel, filtering the gel with a 200-mesh wire gauze, having a pore size of 75 $\mu$m, and drying the gel in a hot air dryer set at 105°C to a constant mass, C (g).

**Patentansprüche**

1. Schmierende Harzzusammensetzung zur persönlichen Pflege, wobei die Zusammensetzung 100 Massenanteile eines thermoplastischen Elastomers mit einer Durometer-A-Härte (HDA) von 10 bis 40, gemessen gemäß dem Japanischen Industriestandard JIS K 7215 (1986), 10 bis 40 Gewichtsanteile eines wasserlöslichen Polyethylenoxids und 20 bis 60 Massenanteile eines wasserabsorbensmodifzierten Polyalkylenoxids umfasst.

2. Schmierende Harzzusammensetzung zur persönlichen Pflege gemäß Anspruch 1, wobei die verwendete Menge an wasserlöslichem Polyethylenoxid (A) und die verwendete Menge an wasserabsorbensmodifziertem Polyalkylenoxid (B) B $\geq$ A erfüllen.

3. Schmierende Harzzusammensetzung zur persönlichen Pflege gemäß Anspruch 1 oder 2, wobei das thermoplastische Elastomer ein styrolbasiertes Elastomer ist.

4. Schmierende Harzzusammensetzung zur persönlichen Pflege gemäß einem der Ansprüche 1 bis 3, wobei das wasserlösliche Polyethylenoxid ein viskositätsmittleres Molekulargewicht, M, von 100.000 bis 5.000.000 aufweist, gemessen gemäß der Formel:

$$[\eta] = K \times M^a$$

worin $[\eta]$ Grenzviskosität darstellt, K ein Koeffizient ist, dessen Wert $6,4 \times 10^{-5}$ (g/dl) ist, und a ein Koeffizient ist, dessen Wert 0,82 ist, wobei die Grenzviskosität $[\eta]$ berechnet werden kann durch Messen spezifischer Viskositäten $\eta_{sp}$ von Lösungen mit unterschiedlichen Polymerkonzentrationen c (g/dl) in reinem Wasser bei 35°C mittels eines Ostwald-Viskosimeters, Teilen der spezifischen Viskosität durch die Polymerkonzentration, um eine reduzierte Viskosität ($\eta^{sp}/C$) zu berechnen, wobei die Grenzviskosität dann basierend auf der Beziehung zwischen reduzierter Viskosität und Polymerkonzentration c ermittelbar ist, wenn die Polymerkonzentration auf 0 extrapoliert wird.

5. Schmierende Harzzusammensetzung zur persönlichen Pflege gemäß einem der Ansprüche 1 bis 4, wobei das wasserabsorbensmodifziertePolyalkylenoxid ein modifiziertes Polyalkylenoxid mit einer Wasserabsorptionsfähigkeit von 10 bis 40 [g/g] ist und durch Reaktion einer Polyalkylenoxidverbindung, einer Diolverbindung und einer Diisocyanatverbindung erhalten wird, wobei die Wasserabsorptionsfähigkeit das Verhältnis B/A (g/g) ist, das ermittelbar ist, indem man etwa 1 g des wasserabsorbensmodifzierten Polyalkylenoxids nimmt, dieses wiegt, um seine Masse, A (g), zu bestimmen, das gewogene modifizierte Polyalkylenoxid in 100 mL an deionisiertem Wasser, abgemessen in einem 200 mL-Becherglas, bei 22°C über 24 Stunden taucht, so dass sich ein Gel bildet, das Gel mit einem 200-Mesh-Drahtgewebe mit einer Porengröße von 75 $\mu$m filtriert und die Masse, B (g), des Gels misst.

6. Schmierende Harzzusammensetzung zur persönlichen Pflege gemäß einem der Ansprüche 1 bis 5, wobei das wasserabsorbensmodifizierte Polyalkylenoxid einen Anteil an einer wasserlöslichen Komponente von 5 bis 30 Massen-% aufweist, wobei der Anteil der wasserlöslichen Komponente [Massen-%] durch die Formel

$$100 \ (A-C)/A$$

angegeben wird und A und C ermittelbar sind, indem man etwa 1 g des wasserabsorbens-modifizierten Polyalkylenoxids nimmt und es wiegt, um seine Masse, A (g), zu bestimmen, das gewogene modifizierte Polyalkylenoxid in 100 mL an deionisiertem Wasser, abgemessen in einem 200 mL-Becherglas, bei 22°C über 24 Stunden taucht, so dass sich ein Gel bildet, das Gel mit einem 200-Mesh Drahtgewebe mit einer Porengröße von 75 $\mu$m filtriert und das Gel in einem Heißlufttrockner, eingestellt bei 105°C, bis zu einer konstanten Masse, C (g), trocknet.

**Revendications**

1. Composition de résine lubrifiante pour l'hygiène personnelle, dans laquelle la composition comprend 100 parties en masse d'un élastomère thermoplastique ayant une dureté au duromètre de type A (HDA) de 10 à 40 mesurée selon la norme Japanese Industrial Standard JIS K 7215 (1986), 10 à 40 parties en masse d'un oxyde de polyéthylène soluble dans l'eau, et 20 à 60 parties en masse d'un oxyde de polyalkylène modifié absorbant l'eau.

2. Composition de résine lubrifiante pour l'hygiène personnelle selon la revendication 1, dans laquelle une quantité utilisée de l'oxyde de polyéthylène soluble dans l'eau (A) et une quantité utilisée de l'oxyde de polyalkylène modifié absorbant l'eau (B) satisfont B $\geq$ A.

3. Composition de résine lubrifiante pour l'hygiène personnelle selon la revendication 1 ou 2, dans laquelle l'élastomère thermoplastique est un élastomère à base de styrène.

4. Composition de résine lubrifiante pour l'hygiène personnelle selon l'une quelconque des revendications 1 à 3, dans laquelle l'oxyde de polyéthylène soluble dans l'eau a une masse moléculaire moyenne en viscosité, M, de 100 000 à 5 000 000, mesurée selon la formule :

$$[\eta] = K \times M^a$$

dans laquelle [n] représente la viscosité limitante, K est un coefficient dont la valeur est 6,4 $\times$ 10$^{-5}$ (g/dl) et a est un coefficient dont la valeur est 0,82, la viscosité limitante [n] pouvant être calculée en mesurant les viscosités spéci-fiques $\eta^{sp}$ de solutions ayant diverses concentrations de polymère c (g/dl) dans l'eau pure à 35 °C à l'aide d'un viscosimètre d'Ostwald, en divisant la viscosité spécifique par la concentration de polymère pour obtenir une viscosité réduite ($n^{sp}$/C), la viscosité limitante pouvant alors être déterminée sur la base de la relation entre la viscosité réduite et la concentration de polymère c, lorsque la concentration de polymère est extrapolée à 0.

5. Composition de résine lubrifiante pour l'hygiène personnelle selon l'une quelconque des revendications 1 à 4, dans laquelle l'oxyde de polyalkylène modifié absorbant l'eau est un oxyde de polyalkylène modifié ayant une capacité d'absorption d'eau de 10 à 40 [g/g] et obtenu par réaction d'un composé d'oxyde de polyalkylène, d'un composé de diol et d'un composé de diisocyanate, dans laquelle la capacité d'absorption d'eau est le rapport B/A (g/g), pouvant être déterminé en prenant environ 1 g de l'oxyde de polyalkylène modifié absorbant l'eau et en le pesant pour trouver sa masse, A (g), en immergeant l'oxyde de polyalkylène modifié pesé dans 100 ml d'eau ayant subi un échange d'ions mesurée dans un bêcher de 200 ml à 22 °C pendant 24 heures pour former un gel, en filtrant le gel à l'aide d'un tamis métallique de 200 mesh, ayant une taille de pore de 75 $\mu$m et en mesurant la masse, B (g), du gel.

6. Composition de résine lubrifiante pour l'hygiène personnelle selon l'une quelconque des revendications 1 à 5, dans laquelle l'oxyde de polyalkylène modifié absorbant l'eau a un rapport d'un composant soluble dans l'eau de 5 à 30 % en masse, dans laquelle le rapport du composant soluble dans l'eau [% en masse] est donné par la formule

$$100 \ (A-C)/A$$

et A et C peuvent être déterminés en prenant environ 1 g de l'oxyde de polyalkylène modifié absorbant l'eau et en le pesant pour trouver sa masse, A (g), en immergeant l'oxyde de polyalkylène modifié pesé dans 100 ml d'eau ayant subi un échange d'ions mesurée dans un bécher de 200 ml à 22 °C pendant 24 heures pour former un gel, en filtrant le gel à l'aide d'un tamis métallique de 200 mesh, ayant une taille de pore de 75 $\mu$m, et en séchant le gel dans un séchoir à air chaud réglé à 105 °C à une masse constante, C (g).

Fig. 1

Width for Monitoring: 20 mm

Fig. 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2010150875 A
- JP 54094961 A
- JP 7024156 A
- JP 9502632 A
- JP 2004509207 A
- JP 2003019372 A

### Non-patent literature cited in the description

- number-average molecular weight. *Japanese Industrial Standards: JIS K,* 2007, 1557-1
- Testing Methods for Durometer Hardness of Plastics. *Japanese Industrial Standard: JIS K 7215,* 1986